# EUROPEAN PATENT SPECIFICATION

(11) **EP 2 549 967 B1**
(45) Date of publication and mention of the grant of the patent: **13.07.2016**
(21) Application number: 11710135.2
(22) Date of filing: 23.03.2011
(51) Int. Cl.: A61F 13/08, D04B 1/14, D04B 1/24, D04B 1/26

(54) **A GARMENT, IN PARTICULAR A COMPRESSION GARMENT FOR MEDICAL USE**
KLEIDUNG, INSBESONDERE MEDIZINISCHE KOMPRESSIONSKLEIDUNG
ARTICLE VESTIMENTAIRE, EN PARTICULIER ARTICLE DE CONTENTION À USAGE MÉDICAL

(30) Priority: 23.03.2010 FR 1052064
(43) Date of publication of application: 30.01.2013
(73) Proprietor: Radiante, 86100 Chatellerault (FR)
(72) Inventor: CLEMENDOT, Olivier, 86530 Naintre (FR)
(74) Representative: Parzy, Benjamin Alain
(86) International application number: PCT/EP2011/001446
(87) International publication number: WO 2011/116952

(56) References cited:
- WO-A1-03/096945
- FR-A- 1 584 803
- JP-A- 2005 029 934

## Description

The present invention relates to garments of the type comprising a main portion to be positioned on a part of the body of a wearer. The garments may notably, but not exclusively, be compression garments for medical use suitable for a leg or an arm. The main portion thereof thus serves to compress the limb on which the garment is positioned, in order to treat lymphatic or venous insufficiency by exerting pressure that is degressive from the bottom towards the top of the garment.

More particularly, the invention relates to garments that are fitted at a welt portion thereof with means for providing an anti-slip effect so as to enhance holding of the garment in place.

### BACKGROUND OF THE INVENTION

Although the question of preventing stockings or socks from slipping down has already given rise to numerous solutions, it should nevertheless be understood that in the special circumstance of compression garments for medical use the problem is more difficult to solve since it is necessary simultaneously to take care to avoid any excessive compression in the welt portion of the garment since that would give rise to a tourniquet effect with a risk of strangulation that is completely undesirable in the medical field, in particular concerning the risk of thrombosis.

During the nineteen seventies, proposals began to be made for anti-slip garter bands added to the welt portion of compression stockings, the anti-slip bands possessing rubberized elements on their inside faces so as to provide the looked-for anti-slip effect. Such bands constituting added elements were assembled by being stitched to the top edge of the main portion of the stocking. For example, reference may be made to document US 3 874 001 A which relates to joining an anti-slip band to the welt portion of a compression stocking while avoiding having stitching that projects excessively on the inside. Proposals have sometimes been made to use bands that concern only a fraction of the circumference of the anti-slip welt portion, as shown in document US 3 975 929 A. Proposals have also been made to coat a localized region of the inside face of the welt portion of a stocking with an anti-friction material so as to obtain small zones of coating that come directly into contact with the wearer's skin, as shown in document US 3 983 870 A. Although that solution might look attractive, it suffers from the drawback of requiring a difficult re-working operation for achieving localized coating in the welt zone of the compression stocking.

During the nineteen eighties, attention was given more to elastic garter bands made of elastic edging ribbon, such bands being coated on the inside with a pattern in the form of strips or pellets made of an anti-slip material such as silicone. Mention may thus be made of document DE 82 17 651 U, which describes an elastic garter band coated on its inside face with silicone strips and/or spots. Nevertheless, in that document, the ribbon extends therapeutic action of the stocking, and as a result runs a risk of strangulation, which makes it relatively ineffective as a medical compression stocking.

In the nineteen nineties, attempts were made above all to improve the structure of the added elastic garter band for the purpose of improving position holding, while always avoiding any risk of strangulation. Reference may be made to document EP 0 621 024 B in the name of the Applicant, which teaches selecting the fibers constituting the braid or the lace of the garter band so that the band both presents an elongation characteristic of shallow slope, and exerts on the limb in question a pressure that is less than the pressure exerted by the main portion in the vicinity of the garter band.

All of the above-mentioned techniques provide for adding a more or less elaborate elastic garter band, but the mere fact of adding automatically implies a separate joining operation that complicates fabrication of the compression garment. It would be very tempting to avoid that constraint by providing a coating of silicone directly on the inside face of the welt portion of the stocking so as to form a circumferential anti-slip band, however that technique would involve an additional fabrication step (like stitching on an additional garter band), and would also make it more complicated to control the pressure exerted at that level.

Certain attempts have subsequently been made to eliminate adding elastic garter bands by attempting to organize the way in which the welt portion is made by knitting using different yarns in order to obtain an anti-slip characteristic, the knitting then taking place without interruption or subsequent re-working, unlike the above-mentioned prior techniques.

The closest state of the art illustrating such an attempt is constituted by document US 6 871 516 B.

That document teaches arranging a band in the end welt portion of a compression stocking, which band is knitted from three different yarns, comprising a cotton or textured nylon body yarn, a locking yarn knitted with the body yarn, made of textured nylon or of covered spandex, and finally a yarn having a high coefficient of friction and made of rubber, neoprene, or of spandex, which yarn is laid into the knitting of the two above-mentioned yarns.

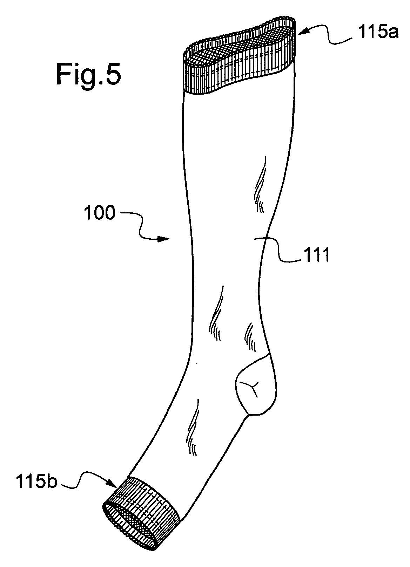

The function of the locking yarn is to guarantee proper positioning of the high-friction yarn, beside the inside face of the zone in question, so as to obtain the looked-for anti-slip effect by the contact area of the laid-in yarns. The drawback of having a laid-in yarn that is the only high-friction element is that the yarn must necessarily be of considerable weight in order to achieve a contact area that is capable of providing the looked-for anti-slip effect (as can be seen in the photographs of Figures 5 and 6) such that, in practice, its weight must always be greater than 300 decitex (dtex), whereas a knitting yarn conventionally has a weight lying in the range 60 dtex to 100 dtex. Nevertheless, such an approach is not satisfactory since any increase in the weight of the high-friction laid-in yarn for increasing contact area with the skin of the wearer of the garment will necessarily have the effect of making the yarn springier and of increasing the pressure exerted on the leg by the zone in question. Under such circumstances, in order to avoid the above-mentioned drawback, and in order to reduce the clamping pressure that is exerted, the only solution consists in spacing apart the rows of laid-in yarn, but that is then detrimental to contact area for a given height of the anti-slip end welt portion. The portion of knit shown in Figure 5 of that document teaches providing a plurality of rows without overlap for the high-friction laid-in yarn, which laid-in yarn is then taken by one needle out of three, thereby having the effect of increasing the contact area of the high-friction laid-in yarn. Nevertheless, that approach is not satisfactory either, since by increasing the number of rows without overlap for the high-friction laid-in yarn, the yarn becomes freer and freer and thus catches more easily and forms undesirable loops.

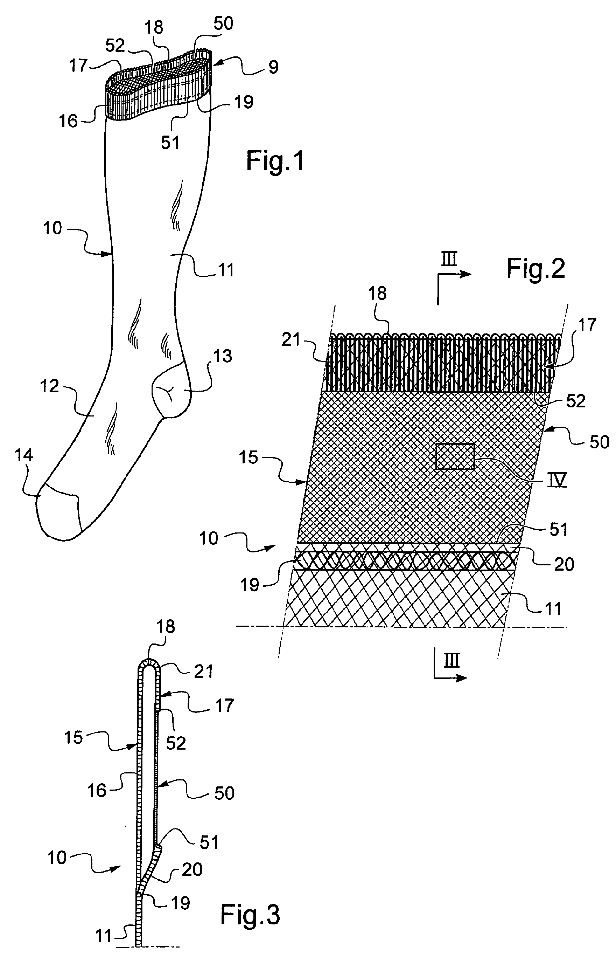

It should be observed that Figure 3 of the above-mentioned document, showing a welt portion with looping performed directly on the circular knitting machine used, is a purely theoretical diagram that is completely unconnected with reality. The high-friction yarn is the laid-in yarn used in the main portion of the compression stocking, and the laid-in yarn is always located on the inside of the knit. When the knit continues in the welt portion , and the end welt portion is folded over with looping of its ends, the high-friction laid-in yarn then lies on the outside and not on the inside of the inside welt of the welt portion , contrary to what is shown in Figure 3.

The attempts shown in document US 6 871 516 B as summarized above have remained purely theoretical in reality since no product implementing that document has been put on the market.

FR 1 584 803 describes another garment of prior art.

### OBJECT OF THE INVENTION

An object of the invention is to devise a garment, in particular but not limited to, a compression garment for medical use having an anti-slip effect arranged at an welt portion thereof in a configuration that offers both to achieve the looked-for anti-slip effect and to avoid any risk of strangulation, and to do so without presenting the above-mentioned drawbacks of the prior documents, in particular those of document US 6 871 516 B described above.

### GENERAL SUMMARY OF THE INVENTION

The technical problem is solved in accordance with the invention by means of a garment as disclosed in claim 1.

Preferably, the anti-slip zone is knitted with the high-friction yarn directly on the knitting machine used for making the main portion so that the anti-slip zone extends on at least a part of an entire circumference of the welt portion, thus procuring a high-friction zone in contact with the wearer's skin.

According to a preferred embodiment, the welt portion is folded so as to define an outside welt and an inside welt, wherein the anti-slip zone is formed on the inside welt.

More particularly, the main portion is made of elastic knit serving to compress the limb on which the garment is positioned, wherein the anti-slip zone is entirely knitted solely with fine high-friction yarn, said anti-slip being knitted directly on the circular knitting machine used for making the main portion and concerning the entire circumference of said inside welt, thus procuring an optimum high-friction zone in contact with the wearer's skin.

Preferably, the high-friction yarn is an elastic yarn, in particular made of spandex or elastodiene. Such a high-friction elastic yarn preferably has a weight lying in the range 50 dtex to 156 dtex. More preferably, the weight of such a high-friction elastic yarn lies in the range 50 dtex to 150 dtex. Even more preferably, the weight of such a high-friction elastic yarn lies in the range 54 dtex to 80 dtex.

Preferably, the anti-slip zone extends nearly on all of a height of the welt portion. The anti-slip zone is at least two centimeters high.

Preferably, one of the outside welt or inside welt has a distal edge that is looped with a looping line of the main portion, the anti-slip zone having a bottom end that extends above the looping line at a distance therefrom. Also, the anti-slip zone has an upper end that extends below a fold of the welt portion, at a distance therefrom.

According to a particular embodiment, the anti-slip zone is made by plain stitch knitting of at least two high-friction yarns. Preferably, the at least two high-friction yarns are of same weight.

In an other particular embodiment, the anti-slip zone is made by plain stitch knitting of the high-friction yarn, the anti-slip zone including an additional high-friction yarn that is knitted with the plain stitch knitted high-friction yarn with 1/n needle selection where n is an integer lying in the range 1 (one needle in two) to 6 (one needle in seven).

In a variant, the plain-stitch knitted high-friction yarn and the additional high-friction yarn are of same weight. In yet another variant, the additional high-friction yarn has a weight greater than a weight of the plain-stitch knitted high-friction yarn.

Preferably, the anti-slip zone is made up of several high-friction yarns that are all of the same kind. Preferably, the anti-slip zone extends uniformly on the entire circumference of the inside welt

In a particularly interesting embodiment, the welt portion is conformed so that it exerts a pressure on the part of the body that is substantially equal to a pressure exerted by the main portion in a vicinity of the welt portion.

Thus, with the invention, and contrary to above-mentioned document US 6 871 516 B in which the anti-slip zone is constituted exclusively by the visible portions of a coarse laid-in yarn, the arrangement of the invention makes it possible to obtain a much greater anti-slip zone by using knitting based solely on high-friction yarns.

The invention also concerns a method of constructing a garment as disclosed in claim 22.

Preferably, the step of integrally-forming the knitted welt comprises the step of forming a tubular knitted welt portion defining an outer face and the inner, skin-contacting face.

Preferably, the step of providing a high-friction yarn comprises the step of providing an elastic high-friction yarn.

Preferably, the step of integrally-forming the knitted welt portion comprises the step of integrally-forming the knitted welt portion uniformly around an entire circumference of the garment.

Preferably, the step of knitting the main garment portion comprises the step of knitting a seamless compression hosiery garment.

Preferably, the step of integrally-forming the knitted welt portion comprises the steps of:
(a)plain stitch knitting a first high-friction yarn;
(b)plain stitch knitting a second high-friction yarn that is knitted with the first high-friction yarn with 1/n needle selection where n is an integer in the range of one needle in two and one needle in seven.

Other characteristics and advantages of the invention appear more clearly in the light of the following description and the drawings showing a particular embodiment.

### BRIEF DESCRIPTION OF THE DRAWINGS

Reference is made to the figures of the accompanying drawings, in which:
- Figure 1 shows a garment in accordance with the invention, here in the form of a compression sock or stocking;
- Figure 2, on a larger scale, shows a portion of the inside periphery of the welt portion of the Figure 1 garment, showing more clearly the anti-slip;
- Figure 3 is a section on III-III of Figure 2, showing more clearly the structure of the welt portion with its anti-slip zone and its end looping;
- Figure 4A is an isolated view on a larger scale of a portion IV of Figure 3 relating to the anti-slip zone, with a conventional type of plain stitch knit;
- Figure 4B shows a variant in which an additional high-friction yarn is used that is knitted with other high-friction yarns with a 1/1 needle selection (every other needle); and
- Figure 4C shows yet another variant, still using an additional high-friction yarn which is knitted with other high-friction yarns, but with a 1/3 needle selection (one needle in four).
- Figure 5 shows yet another garment according to the invention, i.e. an open sock.

### DETAILED DESCRIPTION OF THE PREFERRED EMBODIMENT

Figure 1 shows a garment in accordance with the invention, here implemented in the form of a compression stocking or sock, referenced 10.

The compression garment 10 has a main portion 11 of elastic knit that serves to compress the portion of the leg on which the garment is positioned in order to treat lymphatic or venous insufficiency. The foot portion 12 has a heel 13 and a toe 14. The main portion 11 is also extended upwards by a welt portion also called here top portion referenced 9. The inner face of the top portion 9 in contact with the wearer's skin is conformed to provide an anti-slip effect promoting holding of the compression garment 10 in position. Of course, the inner face of the welt portion is also the inner face of the main portion.

As shown in Figures 1 to 3, the top portion 9 of the compression garment 10 is folded so as to define a folded edge 15 with an outside welt 16 and an inside welt 17 joined together by a top fold 18. A uniform, anti-slip zone 50 is formed on the inside welt 17. The anti-slip zone 50 is entirely knitted solely with fine or low-weight high-friction yarns, the anti-slip zone being knitted directly on the circular knitting machine (not shown herein) used for making the main portion 11 and covering the entire circumference of the inside welt, thus producing an optimum high friction zone in contact with the wearer's skin.

Preferably, the anti-slip zone 50 extends uniformly on the entire circumference of the inside welt 17.

Thus, all of the high-friction yarns knitted in the anti-slip zone 50 contribute to the looked-for anti-slip effect, over the entire knitted pattern. Consequently, for a given area, an optimum contact zone is obtained that is much greater than that that could be obtained using a high-friction laid-in yarn as described in above-mentioned document US 6 871 516 B.

The anti-slip zone 50 extends nearly on all of the height of the inside welt 17 of the folded edge 15. In practice, the height of the anti-slip zone 50 is selected to be equal to at least two centimeters.

The outside welt 16 has a distal edge that is looped with the main portion 11 at a looping line referenced 19. Such a technique of joining by looping is well known in the use of circular knitting machines, with looping then being performed directly on the circular knitting machine without giving rise to extra thicknesses as would happen if connecting stitching were used. In outline, the knitting is modified in the main portion of the garment 11 on reaching the looping line 19 by providing preparatory stitches in the form of loops that are subsequently recovered by the needles when both welts have been knitted in full so as to perform the looping by re-knitting the preparatory stitches.

51 designates the bottom end of the anti-slip zone 50 and 52 designates the upper end of the anti-slip zone 50. Provision is made for the bottom end 51 to extend above and at a distance from the looping line 19, and for the upper end 52 to extend below and at a distance from the top fold 18 of the folded edge 15. Thus, the anti-slip zone 50 is accurately centered in the inside welt 17 of the folded edge 15. Furthermore, because of the bottom intermediate zone 20 that extends between the looping line 19 and the bottom end 51, it is certain there will be no particular disturbance to the knitting of the anti-slip zone 50 during looping. Also because of the top intermediate zone 21 that extends between the top fold 18 and the upper end 52 of the anti-slip zone 50, it is ensured that the anti-slip zone 50 remains facing solely towards the inner face of the folded edge 15. Consequently, the anti-slip zone 50 is always invisible from the outside, where all that can be seen is a small amount of folding in the form of ribs in the corresponding top portion, as shown diagrammatically in Figure 1.

The folded edge 15 is naturally conformed so that the pressure it exerts on the limb is substantially equal to the pressure exerted by the main portion 11 in the vicinity of the folded edge 15, in order to comply with the teaching of above-mentioned document EP 0 621 024 B, so as to avoid any risk of strangulation. This arrangement of the folded edge 15 then applies both to the yarns selected to constitute the inside welt 17 and the outside welt 16, and also naturally to the yarns constituting the anti-slip zone 50, and to the binding technique used.

The anti-slip zone 50 is said to be "uniform" both because of its uniform structure, and because of the regularity of its binding, a fortiori if yarns of the same weight are used, thereby making a genuine high-friction "plane" in contact with the wearer's skin.

There follows a description of various knitting techniques that can be envisaged for implementing the invention, these various techniques sharing in common the exclusive use of fine high-friction yarns. The term "fine" or "low-weight" is used to designate yarns with a weight generally less or equal to 200 dtex. It should be observed that such a weight range is very far from the weight of laid-in high-friction yarns of the kind described in above-mentioned document US 6 871 516 B, which must be greater than at least 300 dtex.

The use of fine yarns makes it possible to have a zone that is completely uniform, which is effective both with respect to the looked-for anti-slip effect, and which is agreeable to the touch because of the continuity of the knitting.

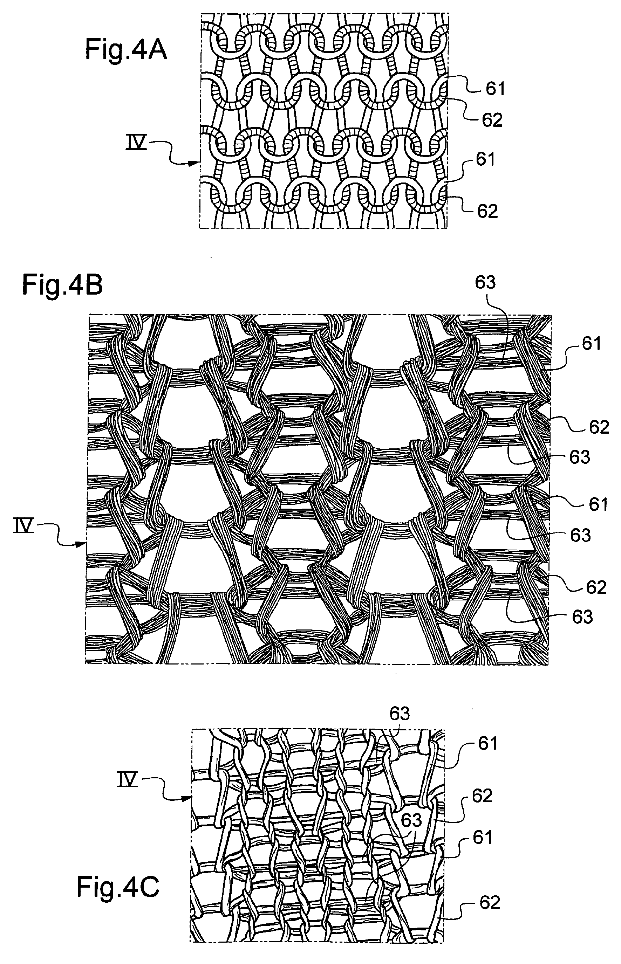

In Figure 4A, there can be seen a portion IV of the anti-slip zone 50, here using a plain stitch knit of one, or better two high-friction yarns referenced 61 and 62. In practice, it is preferable to use two yarns associated with two diametrically-opposite feeds on the circular knitting machine, thus making it possible to make two rows for a given revolution of the cylinder and thus advance twice as fast in fabricating the garment. This naturally constitutes only an example, and provision could equally well be made for more than two yarns to be knitted with plain stitch.

Under all circumstances, it is advantageous for the high-friction yarns 61, 62 that are knitted in plain stitch to have the same weight.

Figure 4B shows a portion IV of the anti-slip zone 50 that also includes an additional high-friction yarn referenced 63. By way of example, this additional high-friction yarn 63 is knitted on a single feed with one or the other of the high-friction yarns 61, 62 of the plain stitch knit, using a needle selection that in this example is of the 1/1 type (i.e. one out of every two needles is taken).

In a variant, as shown in Figure 4C, it is possible to provide for some other needle selection for the additional high-friction yarn 63, e.g. a 1/3 needle selection where one needle in four is taken.

It can be understood on sight of Figures 4B and 4C that the additional yarn 63 that is knitted with one or the other of the yarns 61, 62 may have greater visible portions if the needle selection is increased, however this is limited in practice, since beyond a 1/6 selection (one needle in seven), there is a risk of encountering insufficient retention of the additional high-friction yarn 63, and thus a risk of it catching and forming undesirable loops.

In practice, provision is made for the plain-stitch knitted high-friction yarns 61, 62 and the additional high-friction yarn 63 that is knitted with a selection of needles all to have the same weight.

Nevertheless, in a variant, provision could be made for the high-friction yarns 61 and 62 to be of the same weight, but for the additional high-friction yarn 63 to be of a greater weight, particularly when it is desired to have greater springiness for a higher compression class. Once more, it is appropriate to avoid selecting a weight that is too different since that would be uncomfortable for the wearer.

In any event, the weight of the high-friction yarns is less or equal to 200 dtex.

In general, the high-friction yarns 61, 62, and 63 constituting the anti-slip zone 50 are preferably of the same kind, in particular it is possible to select elastic yarns. Preferably, the high friction yarns are made of spandex or of elastodiene. In that latter case, the weight of the high-friction yarns shall preferably lay between 50 and 156 dtex. The inventors have observed that above 156 dtex, the anti-slip zone 50 is too thight and forms a garrot, which the invention strives to avoid. Below 50 dtex, the anti-slip zone 50 is too loose and does not offer a correct upholding on the covered limb. More preferably, the weight of the high-friction yarns shall lay between 50 and 150 dtex.

Preferably, for elastic yarns in spandex or elastodiene, the weight of the high-friction yarns is between 54 and 80 dtex. The inventors have observed that the anti-slip zone 50 is ideally nervous for upholding the compression garment 10 without impacting the comfort of the wearer. Preferably, for elastic yarns in spandex or elastodiene, the weight of the yarns is substantially 78 dtex.

In the examples show herein, the anti-slip zone does not have any laid-in yarn. Nevertheless, provision could be made for a different arrangement also using laid-in high-friction yarn when knitting on its own is not sufficient to provide a level of compression that is substantially equal to that exerted by the main portion in the vicinity of the folded edge 15.

The invention is not limited to the embodiments described, but covers any variant using equivalent means to reproduce the essential characteristics set out above.

The invention applies to any type of garment the main portion of which being placed of a part of the wearer's body. Thus, the invention generally concerns a garment as disclosed in claim 1. For socks and stockings, it has been found that the invention gives results that are particularly satisfactory with socks (terminating at the top of the calf), since not only is retention naturally enhanced by the shape of the leg (tapering of the calf), but the garment then has no tendency to roll up at the welt portion. The garment may be a glove, a sock, a pantyhose, a stocking, a pantyhose extending to the knee, to the calf, to the ankle, or near the foot end, a brassiere... The main portion thereof may be extended by one or two welt portions according to the invention.

An example of such a garment having two welt portions is given on figure 5 that shows a sock 100 open at the level of the toes. The sock has a main portion 111 in knitting. The main portion 111 is extended by a first welt portion or upper welt portion 115a, and by a second welt portion or lower welt portion 115b. The internal faces of the end portions in contact with the skin of the wearer are preferably conformed for anti-slip purposes promoting holding of the garment 100 in place.

Of course, the invention also applies to garments with no compression effect, for example everyday stockings. Thus, the invention is not limited to a medical use. A person that does not suffer from blood flow or lymphatic problems may wear a garment according to the invention. In addition to protect the leg and the foot, socks and stockings according to the invention are very comfortable because they hold in place without strangulating the leg, like many traditional garments.

Although in the illustrated embodiments the outside welt has a distal edge that is looped to the main portion at a looping line, this may also be performed with the inside welt. In that case, the knitting is modified in the main portion of the garment when arriving at the level of the looping line, by foreseeing some stitches in form of waiting loops. One the outside welt and the inside welt are knitted, the waiting loops are taken over for looping the end of the inside welt by re-knitting of the waiting loops.

Although in the illustrated embodiment, the welt portion is folded so as to define an outside welt and an inside welt, wherein the anti-slip zone is formed on the inside welt, the welt portion could be unfolded. In this case, the anti-slip zone is formed directly on the inner face of the welt portion and extends directly on at least a part of an entire circumference of the welt portion. However, the folded welt portion is preferred as it promotes the retention of the garment in position, whereas an unfolded welt may potentially roll up.

Although in the illustrated embodiment, the anti-slip zone is made by plain stitch knitting of two high friction yarns and an additional high friction yarn, the anti-slip zone could be made differently. For example, the anti-slip zone could be made by plain stitch knitting of a single high friction yarn and an additional high friction yarn.

Although elastics yarns in spandex or elastodiene has been evoked, the yarn or the yarns constituting the anti-slip zone 50 may be in an other material. In particular, several yarns may be used, at least one of which may be elastic.

The choice of the weight range of the yarns of course depends on the nature of the yarn. In any event, the weight of the high-friction yarns is less or equal to 200 dtex, to avoid that the anti-slip zone 50 acts as a garrot.

Although in the illustrated embodiment, the anti-slip zone is knitted directly on a circular knitting machine so that the garment is a seamless compression hosiery garment, the anti-slip zone and the main portion may be knitted on a flat-bed knitting machine or another type of knitting machine.

## Claims

1. A garment (10; 100) including a knitted, circumferentially-extending welt portion having an anti-slip zone (50) that comprises a high friction yarn (61, 62, 63) and that is integrally formed on an inner face of a main garment portion (11; 110) such that the high friction yarn contacts a wearer's skin to increase the anti-slip properties of the garment, wherein the anti-slip zone (50) is entirely knitted solely with fine high-friction yarn (61, 62, 63) having weight less than or equal to 200 dtex.

2. A garment according to claim 1, wherein the anti-slip zone (50) is knitted with the high-friction yarn (61, 62, 63) directly on the knitting machine used for making the main portion (11; 110) so that the anti-slip zone extends on at least a part of a entire circumference of the welt portion, thus procuring a high-friction zone in contact with the wearer's skin.

3. A garment according to claim 2, wherein the welt portion is folded so as to define an outside welt (16) and an inside welt (17), wherein the anti-slip zone is formed on the inside welt.

4. A garment according to claim 3, wherein the main portion (11; 110) is made of elastic knit serving to compress the limb on which the garment is positioned, wherein the anti-slip zone (50) is entirely knitted solely with fine high-friction yarn (61, 62, 63), said anti-slip being knitted directly on the circular knitting machine used for making the main portion (11; 110) and concerning the entire circumference of said inside welt (17), thus procuring an optimum high-friction zone in contact with the wearer's skin.

5. A garment according to claim 1 or claim 4, wherein the high-friction yarn (61, 62, 63) is an elastic yarn.

6. A garment according to claim 5, wherein the high-friction yarn (61, 62, 63) is made of spandex or elastodiene.

7. A garment according to claim 6, wherein the weight of the high-friction yarn (61, 62, 63) lies in the range 50 dtex to 156 dtex.

8. A garment according to claim 6, wherein the weight of the high-friction elastic yarn (61, 62, 63) lies in the range 50 dtex to 150 dtex.

9. A garment according to claim 6, wherein the high-friction elastic yarn (61, 62, 63) lies in the range 54 dtex to 80 dtex.

10. A garment according to claim 1 or 4, wherein the anti-slip zone (50) extends nearly on all of a height of the welt portion.

11. A garment according to claim 1 or 4, wherein the anti-slip zone (50) is at least two centimeters high.

12. A garment according to claim 3 or 4, wherein one of the outside welt (16) or inside welt (17) has a distal edge that is looped with a looping line (19) of the main portion (11; 110), the anti-slip zone having a bottom end (51) that extends above the looping line at a distance therefrom.

13. A garment according to claim 3 or 4, wherein the anti-slip zone (50) has a upper end (52) that extends below a fold of the welt portion, at a distance therefrom.

14. A garment according to claim 1 or 4, wherein the anti-slip zone (50) is made by plain stitch knitting of at least two high-friction yarns (61, 62, 63).

15. A garment according to claim 14, wherein the at least two high-friction yarns (61, 62, 63) are of same weight.

16. A garment according to claim 1 or 4, wherein the anti-slip zone (50) is made by plain stitch knitting of the high-friction yarn (61, 62), the anti-slip zone including an additional high-friction yarn (63) that is knitted with the plain stitch knitted high-friction yarn with 1/n needle selection where n is an integer lying in the range 1 (one needle in two) to 6 (one needle in seven).

17. A garment according to claim 16, wherein the plain-stitch knitted high-friction yarn (61, 62) and the additional high-friction yarn (63) are of same weight.

18. A garment according to claim 16, wherein the additional high-friction yarn (63) has a weight greater than a weight of the plain-stitch knitted high-friction yarn (61, 62).

19. A garment according to claim 1 or 4, wherein the anti-slip zone (50) is made up of several high-friction yarns (61, 62, 63) that are all of the same kind.

20. A garment according to claim 1 or 4, wherein the welt portion is conformed so that it exerts a pressure on the part of the body that is substantially equal to a pressure exerted by the main portion in a vicinity of the welt portion.

21. A garment according to claim 1 or claim 4, wherein the anti-slip zone (50) extends uniformly on the entire circumference of the inside welt (17).

22. A method of constructing a garment (10, 100) having anti-slip properties for maintaining the garment in a desired position on a wearer's body, comprising the steps of:
(a) knitting a main garment portion (11; 110) adapted to positioned onto the body of the wearer;
(b) providing fine high-friction yarn (61, 62, 63) having weight less than or equal to 200 dtex; and
(c) integrally-forming a knitted welt portion including the fine high-friction yarn circumferentially onto an inner, skin-contacting face of the knitted welt portion, such that the anti-slip zone (50) is entirely knitted solely with fine high-friction yarn (61, 62, 63) having weight less than or equal to 200 dtex, to define an anti-slip zone such that the high friction yarn contacts a wearer's skin to increase the anti-slip properties of the garment.

23. A method according to claim 22, wherein the step of integrally-forming the knitted welt comprises the step of forming a tubular knitted welt portion defining an outer face and the inner, skin-contacting face.

24. A method according to claim 22, wherein the step of providing a high-friction yarn (61, 62, 63) comprises the step of providing an elastic high-friction yarn.

25. A method according to claim 22, wherein the step of integrally-forming the knitted welt portion comprises the step of integrally-forming the knitted welt portion uniformly around an entire circumference of the garment.

26. A method according to claim 22, wherein the step of knitting the main garment portion (11, 111) comprises the step of knitting a seamless compression hosiery garment.

27. A method according to claim 22, wherein the step of integrally-forming the knitted welt portion comprises the steps of:
(a) plain stitch knitting a first high-friction yarn (61, 62);
(b) plain stitch knitting a second high-friction yarn (63) that is knitted with the first high-friction yarn with 1/n needle selection where n is an integer in the range of one needle in two and one needle in seven.

## Patentansprüche

1. Kleidungsstück (10; 100), das einen gestrickten, sich in Umfangsrichtung erstreckenden Bundabschnitt mit einer Antirutschzone (50) enthält, die ein reibungsstarkes Garn (61, 62, 63) umfasst und die auf einer Innenfläche eines Hauptkleidungsstückabschnittes (11; 110) derart integral ausgebildet ist, dass das reibungsstarke Garn die Haut eines Trägers kontaktiert, um die Antirutscheigenschaften des Kleidungsstückes zu erhöhen, wobei die Antirutschzone (50) vollständig einzig aus feinem reibungsstarkem Garn (61, 62, 63) mit einem Gewicht von kleiner oder gleich 200 dtex gestrickt ist.

2. Kleidungsstück nach Anspruch 1, wobei die Antirutschzone (50) aus dem reibungsstarken Garn (61, 62, 63) direkt auf der Strickmaschine gestrickt wird, die zum Herstellen des Hauptabschnittes (11; 110) verwendet wird, so dass sich die Antirutschzone auf mindestens einem Teil eines gesamten Umfangs des Bundabschnittes erstreckt und somit eine reibungsstarke Zone in Kontakt mit der Haut des Trägers bereitstellt.

3. Kleidungsstück nach Anspruch 2, wobei der Bundabschnitt umgelegt ist, um einen äußeren Bund (16) und einen inneren Bund (17) zu definieren, wobei die Antirutschzone auf dem inneren Bund ausgebildet ist.

4. Kleidungsstück nach Anspruch 3, wobei der Hauptabschnitt (11; 110) aus elastischem Gestrick hergestellt ist, das dazu dient, die Extremität, auf der das Kleidungsstück positioniert ist, zu komprimieren, wobei die Antirutschzone (50) vollständig einzig aus feinem reibungsstarkem Garn (61, 62, 63) gestrickt ist, wobei die Antirutschzone direkt auf der Rundstrickmaschine gestrickt wird, die zum Herstellen des Hauptabschnittes (11; 110) verwendet wird, und den gesamten Umfang des inneren Bundes (17) betrifft und somit eine optimale reibungsstarke Zone in Kontakt mit der Haut des Trägers bereitstellt.

5. Kleidungstück nach Anspruch 1 oder Anspruch 4, wobei das reibungsstarke Garn (61, 62, 63) ein elastisches Garn ist.

6. Kleidungsstück nach Anspruch 5, wobei das reibungsstarke Garn (61, 62, 63) aus Elastan oder Elastodien hergestellt ist.

7. Kleidungsstück nach Anspruch 6, wobei das Gewicht des reibungsstarken Garns (61, 62, 63) im Bereich 50 dtex bis 156 dtex liegt.

8. Kleidungsstück nach Anspruch 6, wobei das Gewicht des reibungsstarken elastischen Garns (61, 62, 63) im Bereich 50dtex bis 150 dtex liegt.

9. Kleidungsstück nach Anspruch 6, wobei das reibungsstarke elastische Garn (61, 62, 63) im Bereich 54 dtex bis 80 dtex liegt.

10. Kleidungsstück nach Anspruch 1 oder 4, wobei sich die Antirutschzone (50) nahezu über eine gesamte Höhe des Bundabschnittes erstreckt.

11. Kleidungsstück nach Anspruch 1 oder 4, wobei die Antirutschzone (50) mindestens zwei Zentimeter hoch ist.

12. Kleidungsstück nach Anspruch 3 oder 4, wobei entweder der äußere Bund (16) oder der innere Bund (17) eine distale Kante hat, die mit einer Schlingenlinie (19) des Hauptabschnittes (11; 110) verschlungen ist, wobei die Antirutschzone ein unteres Ende (51) hat, das sich über der Schlingenlinie in einem Abstand zu derselben erstreckt.

13. Kleidungsstück nach Anspruch 3 oder 4, wobei die Antirutschzone (50) ein oberes Ende (52) hat, das sich unter einer Falte des Bundabschnittes in einem Abstand zu derselben erstreckt.

14. Kleidungsstück nach Anspruch 1 oder 4, wobei die Antirutschzone (50) durch Stricken von Rechtsmaschen aus mindestens zwei reibungsstarken Garnen (61, 62, 63) hergestellt ist.

15. Kleidungsstück nach Anspruch 14, wobei die mindestens zwei reibungsstarken Garne (61, 62, 63) das gleiche Gewicht haben.

16. Kleidungsstück nach Anspruch 1 oder 4, wobei die Antirutschzone (50) durch Stricken von Rechtsmaschen mit dem reibungsstarken Garn (61, 62) hergestellt ist, wobei die Antirutschzone ein zusätzliches reibungsstarkes Garn (63) enthält, das mit dem rechts gestrickten reibungsstarken Garn mit einer 1/n Nadelauswahl gestrickt wird, wobei n eine ganze Zahl ist, die im Bereich 1 (jede zweite Nadel) bis 6 (jede siebte Nadel) liegt.

17. Kleidungsstück nach Anspruch 16, wobei das rechts gestrickte reibungsstarke Garn (61, 62) und das zusätzliche reibungsstarke Garn (63) das gleiche Gewicht haben.

18. Kleidungsstück nach Anspruch 16, wobei das zusätzliche reibungsstarke Garn (63) ein Gewicht hat, das größer als ein Gewicht des rechts gestrickten reibungsstarken Garns (61, 62) ist.

19. Kleidungsstück nach Anspruch 1 oder 4, wobei die Antirutschzone (50) aus mehreren reibungsstarken Garnen (61, 62, 63) besteht, die alle gleicher Art sind.

20. Kleidungsstück nach Anspruch 1 oder 4, wobei der Bundabschnitt so angepasst ist, dass er einen Druck auf den Teil des Körpers ausübt, der im Wesentlichen gleich einem Druck ist, der von dem Hauptabschnitt in einer Nähe des Bundabschnittes ausgeübt wird.

21. Kleidungsstück nach Anspruch 1 oder Anspruch 4, wobei sich die Antirutschzone (50) gleichmäßig über den gesamten Umfang des inneren Bundes (17) erstreckt.

22. Verfahren zum Herstellen eines Kleidungsstückes (10, 100) mit Antirutscheigenschaften zum Halten des Kleidungsstückes in einer gewünschten Position auf dem Körper eines Trägers, umfassend die Schritte:
(a) Stricken eines Hauptkleidungsstückabschnittes (11; 110), der daran angepasst ist, auf dem Körper des Trägers positioniert zu werden;
(b) Bereitstellen von feinem reibungsstarkem Garn (61, 62, 63) mit einem Gewicht von kleiner oder gleich 200 dtex; und
(c) integrales Ausbilden eines gestrickten Bundabschnittes, der das feine reibungsstarke Garn in Umfangsrichtung auf einer inneren, die Haut kontaktierenden Seite des gestrickten Bundabschnittes enthält derart, dass die Antirutschzone (50) vollständig einzig mit feinem reibungsstarkem Garn (61, 62, 63) mit einem Gewicht von kleiner oder gleich 200 dtex gestrickt wird, um eine Antirutschzone derart zu definieren, dass das reibungsstarke Garn eine Haut eines Trägers kontaktiert, um die Antirutscheigenschaften des Garns zu erhöhen.

23. Verfahren nach Anspruch 22, wobei der Schritt des integralen Ausbildens des gestrickten Bundes den Schritt des Ausbildens eines schlauchförmigen gestrickten Bundabschnittes umfasst, der eine äußere Seite und die innere, die Haut kontaktierende Seite definiert.

24. Verfahren nach Anspruch 22, wobei der Schritt des Bereitstellens eines reibungsstarken Garns (61, 62, 63) den Schritt des Bereitstellens eines elastischen reibungsstarken Garns umfasst.

25. Verfahren nach Anspruch 22, wobei der Schritt des integralen Ausbildens des gestrickten Bundabschnittes den Schritt des integralen Ausbildens des gestrickten Bundabschnittes gleichmäßig um einen gesamten Umfang des Kleidungsstückes herum umfasst.

26. Verfahren nach Anspruch 22, wobei der Schritt des Strickens des Hauptkleidungsstückabschnittes (11, 111) den Schritt des Strickens eines nahtlosen Kompressionsstrumpfkleidungsstückes umfasst.

27. Verfahren nach Anspruch 22, wobei der Schritt des integralen Ausbildens des gestrickten Bundabschnittes die Schritte umfasst:
(a) Stricken rechter Maschen aus einem ersten reibungsstarken Garn (61, 62);
(b) Stricken rechter Maschen aus einem zweiten reibungsstarken Garn (63), das mit dem ersten reibungsstarken Garn mit einer 1/n Nadelauswahl gestrickt wird, wobei n eine ganze Zahl im Bereich zwischen jeder zweiten Nadel und jeder siebten Nadel ist.

## Revendications

1. Article vestimentaire (10 ; 100) comprenant une portion de bordure tricotée s'étendant circonférentiellement comportant une zone d'anti-glissement (50) qui comprend un fil à haut coefficient de friction (61, 62, 63) et qui est intégralement formée sur une face interne d'une partie principale de l'article vestimentaire (11 ; 110) de façon à ce que le fil à haut coefficient de friction soit en contact avec la peau d'un porteur pour augmenter les propriétés d'anti-glissement de l'article vestimentaire, dans lequel la zone d'anti-glissement (50) est entièrement tricotée uniquement avec un fil de faible titre à haut coefficient de friction (61, 62, 63) ayant un titre inférieur ou égal à 200 dtex.

2. Article vestimentaire selon la revendication 1, dans lequel la zone d'anti-glissement (50) est tricotée avec le fil à haut coefficient de friction (61, 62, 63) directement sur le métier à tricoter utilisé pour réaliser la partie principale (11 ; 110) de telle sorte que la zone d'anti-glissement s'étende sur au moins une partie de la totalité de la circonférence de la portion de bordure procurant ainsi une zone à haut coefficient de friction en contact avec la peau du porteur.

3. Article vestimentaire selon la revendication 2, dans lequel la portion de bordure est repliée afin de former une bordure extérieure (16) et une bordure intérieure (17), la zone d'anti-glissement étant formée sur la bordure intérieure.

4. Article vestimentaire selon la revendication 3, dans lequel la partie principale (11 ; 110) est constituée d'un tricot élastique servant à comprimer le membre sur lequel l'article vestimentaire est positionné, la zone d'anti-glissement (50) étant entièrement tricotée uniquement avec un fil à faible titre à haut coefficient de friction (61, 62, 63), ladite zone d'anti-glissement étant tricotée directement sur le métier circulaire utilisé pour réaliser la partie principale (11 ; 110) et concernant la totalité de la circonférence de ladite bordure intérieure (17), procurant ainsi une zone à haut coefficient de friction optimum en contact avec la peau du porteur.

5. Article vestimentaire selon la revendication 1 ou la revendication 4, dans lequel le fil à haut coefficient de friction (61, 62, 63) est un fil élastique.

6. Article vestimentaire selon la revendication 5, dans lequel le fil à haut coefficient de friction (61, 62, 63) se compose de spandex ou d'élastodiène.

7. Article vestimentaire selon la revendication 6, dans lequel le titre du fil à haut coefficient de friction (61, 62, 63) est compris entre 50 dtex et 156 dtex.

8. Article vestimentaire selon la revendication 6, dans lequel le titre du fil élastique à haut coefficient de friction (61, 62, 63) est compris entre 50 dtex et 150 dtex.

9. Article vestimentaire selon la revendication 6, dans lequel le fil élastique à haut coefficient de friction (61, 62, 63) a un titre compris entre 54 dtex et 80 dtex.

10. Article vestimentaire selon la revendication 1 ou 4, dans lequel la zone d'anti-glissement (50) s'étend sur la presque totalité d'une hauteur de la portion de bordure.

11. Article vestimentaire selon la revendication 1 ou 4, dans lequel la zone d'anti-glissement (50) mesure au moins 2 centimètres de haut.

12. Article vestimentaire selon la revendication 3 ou 4, dans lequel l'une de la bordure extérieure (16) ou de la bordure intérieure (17) a une extrémité qui est remmaillée avec une ligne de remmaillage (19) de la partie principale (11 ; 110), la zone d'anti-glissement comportant un bord inférieur (51) qui s'étend au-dessus et à distance de la ligne de remmaillage.

13. Article vestimentaire selon la revendication 3 ou 4, dans lequel la zone d'anti-glissement (50) comporte un bord supérieur (52) qui s'étend en dessous et à distance d'un pli de la portion de bordure.

14. Article vestimentaire selon la revendication 1 ou 4, dans lequel la zone d'anti-glissement (50) est réalisée par un tricotage en maille jersey d'au moins deux fils à haut coefficient de friction (61, 62, 63).

15. Article vestimentaire selon la revendication 14, dans lequel lesdits au moins deux fils à haut coefficient de friction (61, 62, 63) sont de même titre.

16. Article vestimentaire selon la revendication 1 ou 4, dans lequel la zone d'anti-glissement (50) est réalisée par un tricotage en maille jersey du fil à haut coefficient de friction (61, 62), la zone d'anti-glissement comprenant un fil à haut coefficient de friction supplémentaire (63) qui est tricoté avec le fil à haut coefficient de friction tricoté en maille jersey avec sélection d'aiguilles 1/n, où n est un nombre entier allant de 1 (1 aiguille sur 2) à 6 (1 aiguille sur 7).

17. Article vestimentaire selon la revendication 16, dans lequel le fil à haut coefficient de friction tricoté en maille jersey (61, 62) et le fil à haut coefficient de friction supplémentaire (63) sont de même titre.

18. Article vestimentaire selon la revendication 16, dans lequel le fil à haut coefficient de friction supplémentaire (63) a un titre supérieur à un titre du fil à haut coefficient de friction tricoté en maille jersey (61, 62).

19. Article vestimentaire selon la revendication 1 ou 4, dans lequel la zone d'anti-glissement (50) est constituée de plusieurs fils à haut coefficient de friction (61, 62, 63) qui sont tous de même nature.

20. Article vestimentaire selon la revendication 1 ou 4, dans lequel la portion de la bordure est conformée de telle sorte qu'elle exerce une pression sur la partie du corps qui est sensiblement égale à une pression exercée par la partie principale au voisinage de la portion de bordure.

21. Article vestimentaire selon la revendication 1 ou la revendication 4, dans lequel la zone d'anti-glissement (50) s'étend uniformément sur la totalité de la circonférence de la bordure intérieure (17).

22. Procédé de fabrication d'un article vestimentaire (10, 100) ayant des propriétés d'anti-glissement pour maintenir l'article vestimentaire dans une position souhaitée sur le corps d'un porteur, comprenant les étapes de :
(a) tricoter une partie principale de l'article vestimentaire (11 ; 110) conçue pour un positionnement sur le corps du porteur ;
(b) fournir un fil de faible titre à haut coefficient de friction (61, 62, 63) ayant un titre inférieur ou égal à 200 dtex ; et
(c) former intégralement une portion de bordure tricotée comprenant le fil de faible titre à haut coefficient de friction, circonférentiellement, sur une face interne de la portion de bordure en contact avec la peau, de façon à ce que la zone d'anti-glissement (50) soit entièrement tricotée uniquement avec un fil de faible titre à haut coefficient de friction (61, 62, 63) ayant un titre inférieur ou égal à 200 dtex, pour former une zone d'anti-glissement de façon à ce que le fil à haut coefficient de friction soit en contact avec la peau d'un porteur pour augmenter les propriétés d'anti-glissement de l'article vestimentaire.

23. Procédé selon la revendication 22, dans lequel l'étape de formation en une seule pièce de la bordure tricotée comprend l'étape de formation d'une portion de bordure tricotée tubulaire formant une face externe et la face interne, en contact avec la peau.

24. Procédé selon la revendication 22, dans lequel l'étape de fourniture d'un fil à haut coefficient de friction (61, 62, 63) comprend l'étape de fourniture d'un fil élastique à haut coefficient de friction.

25. Procédé selon la revendication 22, dans lequel l'étape de formation en une seule pièce de la portion de bordure tricotée comprend l'étape de formation en une seule pièce de la portion de bordure tricotée, uniformément, sur la totalité d'une circonférence de l'article vestimentaire.

26. Procédé selon la revendication 22, dans lequel l'étape de tricotage de la partie principale de l'article vestimentaire (11, 111) comprend l'étape de tricotage d'un article de contention sans couture.

27. Procédé selon la revendication 22, dans lequel l'étape de formation en une seule pièce de la portion de bordure tricotée comprend les étapes de :
(a) tricoter en maille jersey un premier fil à haut coefficient de friction (61, 62) ;
(b) tricoter en maille jersey un deuxième fil à haut coefficient de friction (63) qui est tricoté avec le premier fil à haut coefficient de friction avec sélection d'aiguilles 1/n, où n est un nombre entier allant de 1 aiguille sur 2 à 1 aiguille sur 7.
